(19) Europäisches Patentamt
European Patent Office
Office européen des brevets

(11) Publication number: **0 482 481 A1**

(12) **EUROPEAN PATENT APPLICATION**

(21) Application number: **91117599.0**

(22) Date of filing: **16.10.91**

(51) Int. Cl.[5]: **C07D 471/14**, //(C07D471/14, 243:00,221:00,221:00)

(30) Priority: **19.10.90 US 600451**

(43) Date of publication of application: **29.04.92 Bulletin 92/18**

(84) Designated Contracting States: **AT BE CH DE DK ES FR GB GR IT LI LU NL SE**

(71) Applicant: **Boehringer Ingelheim Pharmaceuticals Inc.**
**90 East Ridge P.O. Box 368**
**Ridgefield Connecticut 06877(US)**

(72) Inventor: **Grozinger, Karl G.**
**171 High Ridge**
**Ridgefield, CT 06877(US)**
Inventor: **Hargrave, Karl D.**
**4 Edna Drive**
**Brookfield, CT 06805(US)**
Inventor: **Adams, Julian**
**270 Peaceable Street**
**Ridgefield, CT 06877(US)**

(74) Representative: **Laudien, Dieter, Dr. et al**
**Boehringer Ingelheim Zentrale GmbH ZA**
**Patente Postfach 200**
**W-6507 Ingelheim am Rhein(DE)**

(54) **Method for the preparation of 5,11-dihydro-6H-dipyrido[3,2-b:2',3'-e][1,4]diazepines.**

(57) A method for preparing certain 5,11-dihydro-6H-dipyrido(3,2-b:2',3'-e][1,4]diazepines which comprises the following steps:

1. converting a 2,6-dichloro-3-pyridinecarboxamide to a 2,6-dichloro-3-aminopyridine by Hofmann Rearrangement;
2. reacting the 2,6-dichloro-3-aminopyridine with a 2-chloronicotonic acid to produce a 2,6-dichloro-3-(2'-chloro-3'-nicotinyl)carboxamidopyridine;
3. reacting the 2,6-dichloro-3-(2'-chloro-3'-nicotinyl) carboxamidopyridine with an amine to produce a 2,6-dichloro-3-(2'-amino-3'-nicotinyl)carboxamidopyridine; and
4. hydrogenating the 2,6-dichloro-3-(2'-amino-3'-nicotinyl)carboxamidopyridine to produce the 5,11-dihydro-6H-dipyrido[3,2-b:2',3'-e][1,4]diazepine.

EP 0 482 481 A1

FIELD OF THE INVENTION

This invention relates to a novel method for preparing certain 5,11-dihydro-6H-dipyrido[3,2-b:2',3'-e]-[1,4]diazepines.

BACKGROUND OF THE INVENTION

Copending Philippine Patent Application No. 42529 describe novel 5,11-dihydro-6H-dipyrido[3,2-b:2',3'-e][1,4]diazepines useful in the prevention and treatment of HIV infection and methods for preparing these compounds.

SUMMARY OF THE INVENTION

Pyridobenzodiazepine compounds prepared by the novel process of this invention and from the novel intermediates described herein have the formula:

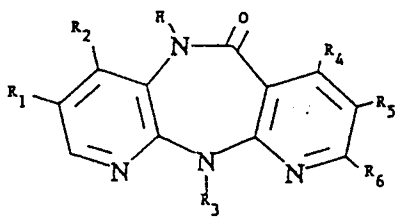

(I)

wherein $R_1$ is hydrogen, alkyl of 1 to 4 carbon atoms, alkoxy or alkylthio of 1 to 4 carbon atoms, alkoxycarbonyl of 2 to 4 carbon atoms, trihalomethyl, hydroxyalkyl of 1 to 4 carbon atoms, alkanoyl of 2 to 4 carbon atoms, alkanoyloxy of 2 to 4 carbon atoms, alkanoylamino of 1 to 4 carbon atoms, aminoalkyl of 1 to 4 carbon atoms, alkoxycarbonylalkyl wherein the alkoxy and alkyl moieties each contain 1 to 2 carbon atoms, carboxyalkyl of 2 to 4 carbon atoms, mono- or di-alkylamino wherein each alkyl moiety contains 1 to 2 carbon atoms, cyano, nitro, hydroxyl, carboxyl, amino, mono- or di-alkylaminoalkyl wherein each alkyl moiety contains 1 to 2 carbon atoms, azido or halogen:

$R_2$ is hydrogen or alkyl of 1 to 3 carbon atoms, with the proviso that if $R_1$ is other than hydrogen or alkyl of 1 to 3 carbon atoms, then $R_2$ is hydrogen;

$R_3$ is alkyl or fluoroalkyl of 1 to 5 carbon atoms, cycloalkyl of 3 to 6 carbon atoms, alkEnyl or alkynyl of 2 to 5 carbon atoms, alkoxyalkyl or alkylthioalkyl of 2 to 4 carbon atoms, alkanoyl of 2 to 4 carbon atoms, hydroxyalkyl of 2 to 5 carbon atoms, preferably 2 to 4 carbon atoms, arylmethyl (wherein the aryl moiety is phenyl, thienyl or furanyl, which is either unsubstituted or substituted by alkyl or alkoxy of 1 to 3 carbon atoms, hydroxyl or halogen), phenyl (which is either unsubstituted or substituted by alkyl or alkoxy of 1 to 3 carbon atoms, hydroxy or halogen) or alkoxycarbonylmethyl wherein the alkoxy moiety contains 1 to 5 carbon atoms;

one of $R_4$, $R_5$, and $R_6$ is alkyl of 1 to 4 carbon atoms, alkenyl or alkynyl of 2 to 4 carbon atoms, trihalomethyl, hydroxyalkyl of 1 to 4 carbon atoms, alkyloxyalkyl or alkylthioalkyl of 2 to 4 carbon atoms, alkyloxycarbonylalkyl wherein the alkyl moieties each contain 1 to 2 carbon atoms, hydroxyl, alkyloxy or alkylthio of 1 to 4 carbon atoms, hydroxyalkyloxy of 2 to 4 carbon atoms, alkanoyloxy of 2 to 4 carbon atoms, alkylsulfinyl or alkylsulfonyl of 1 to 4 carbon atoms, alkanoyl of 2 to 6 carbon atoms, alkoxycarbonyl wherein the alkyl moiety contains 1 to 3 carbon atoms, aminoalkyl of 1 to 4 carbon atoms, mono- or di-alkylaminoalkyl wherein each alkyl moiety contains 1 to 2 carbon atoms, halogen, cyano, nitro, azido or carboxyl, with the other two substituents being hydrogen;

or, two of $R_4$, $R_5$ and $R_6$ are independently alkyl of 1 to 2 carbon atoms, trihalomethyl, alkyloxy or alkylthio of 1 to 2 carbon atoms, or halogen, with the remaining substituent being hydrogen;

or $R_4$, $R_5$ and $R_6$ are each hydrogen.

Preferably, $R_1$ and $R_2$ are hydrogen or alkyl of 1 to 3 carbon atoms, and $R_3$ is alkyl of 1 to 4 carbon atoms or cycloalkyl of 3 to 6 atoms.

More preferably, $R_1$ and $R_2$ are hydrogen or alkyl of 1 to 3 carbon atoms; $R_3$ is cycloalkyl of 3 to 6 carbon atoms; and $R^4$, $R^5$, and $R^6$ are each hydrogen.

The process of this invention for the preparation of the compound of formula I is outlined below:

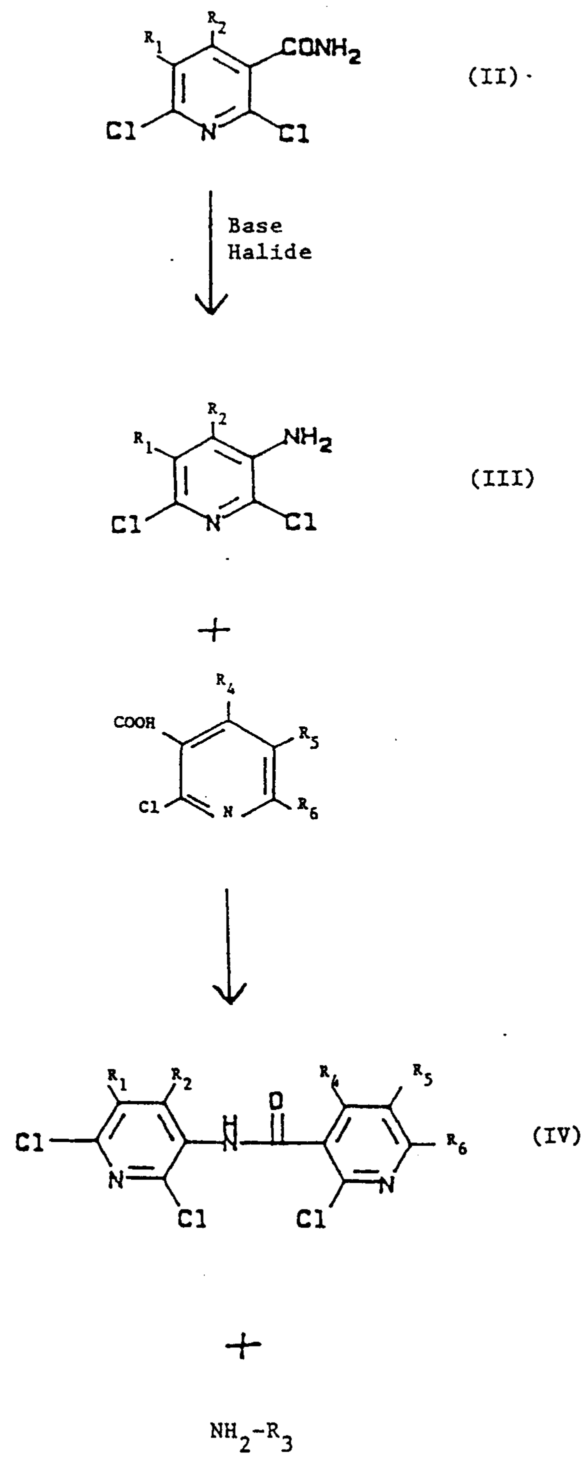


(II)

(III)

(IV)

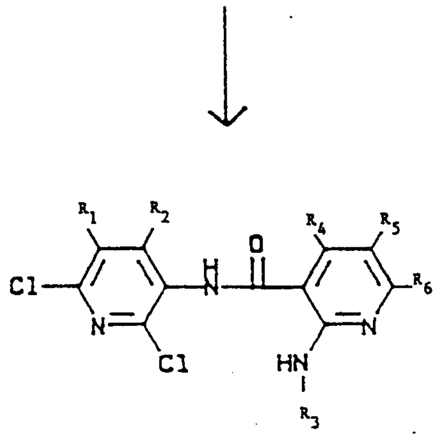

(V)

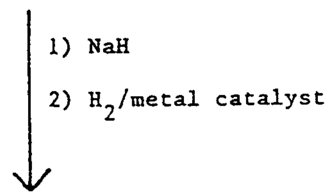


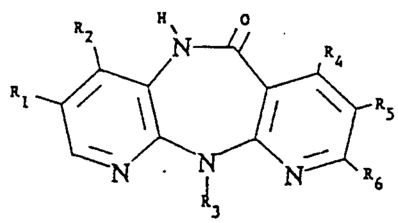

(I)

DETAILED DESCRIPTION OF THE INVENTION

The novel process of this invention for preparing the pyridobenzodiazepine of formula I comprises the following steps:

Step 1, reacting a compound having the formula

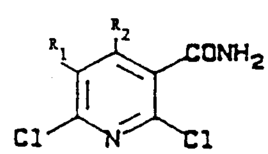

(II)

wherein $R_1$ and $R_2$ are as defined above, in an agueous solution, with a base, such as NaOH, and a halide, such as bromine, chlorine or NaOBr, at temperature ranging from about 0 ° C to about 100 ° C, to produce a compound having the formula

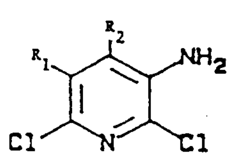

(III)

$R_1$ and $R_2$ have their starting values;

Step 2, reacting the compound produced in Step 1 with a compound of the formula

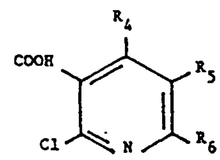

wherein $R_4$, $R_5$ and $R_6$ are as defined above, in an anhydrous organic solvent, such as pyridine, between about 20 ° C to about 80 ° C (preferably about 70 ° C), to produce a compound having the formula

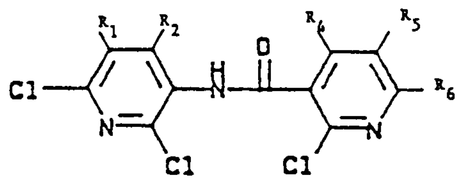

(IV)

wherein $R_1$, $R_2$, $R_4$, $R_5$ and $R_6$ are the same as in the starting reactants;

Step 3, reacting the compound produced in Step 2 with $R_3NH_2$, wherein $R_3$ is as defined above, in an inert organic solvent, such as diglyne or xylene, between about 20 ° C to about 150 ° C (preferably about 90 ° to about 110 ° C), to produce a compound of the formula

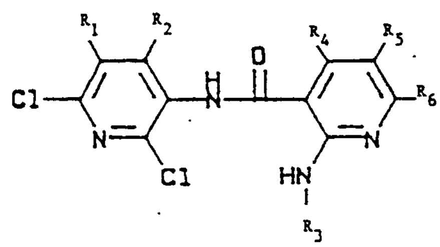

(V)

wherein $R_1$ - $R_6$ are the same as in the starting reactants; and

Step 4, heating the compound produced in Step 3 in an inert organic solvent, such as pyridine, DMF or diglyne, comprising sodium hydride, between about 60 ° C to about 150 ° C; then hydrogenating the resultant solution in the presence of a metal catalyst, such as palladium, between about 20 ° C to about 100 ° C, to produce a mixture containing the compound of formula I; and then isolating the compound of formula I from the mixture.

Preparation A below illustrates the preparation of the 2,6-dichloro-3-pyridinecarboxamide (compound of formula II) used to prepare the pyridobenzodiazepinone of Example I.

The compound may be isolated as such and, if desired, converted into a salt, e.g. an acid addition salt, or isolated from the mixture as a salt; known methods can be used for forming the salts.

The following Examples illustrate the preparation of various pyridobenzodiazepinones of formula I.

PREPARATION A

1) PREPARATION OF 3-CYANO-2,6-DIHYDRO-4-METHYLPYRIDINE

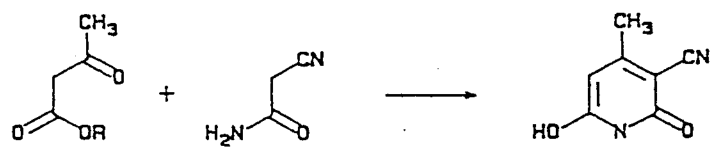

R = Ethyl

A mixture of 336g (4 moles) of cyanoacetamide, 507ml (520g, 4 moles) of ethyl acetoacetate, and 850ml of methanol was warmed to attain solution and 275g (4.18 moles) of potassium hydroxide dissolved in 220ml of methanol was added during 2 hours with stirring. During the addition a white precipitate formed and more methanol was added to prevent caking. The mixture was heated to reflux, stirred for 8 hours, cooled and filtered. The white precipitate was washed with methanol. The mono potassium salt was dissolved in warm water, filtered, cooled, acidified with concentrated hydrochloric acid, filtered, washed with water, and dried at 90°C to yield 535g (89%).

2) PREPARATION OF 3-CYANO-2,6-DICHLORO-4-METHYLPYRIDINE

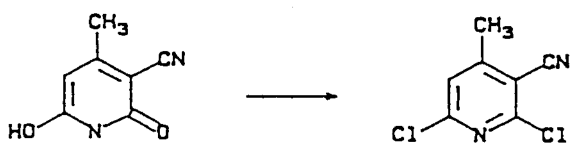

3-Cyano-2,6-dihydroxy-4-methylpyridine (50g, 0.33 mole) and phosphorous oxychloride (100ml, 1.07 mole) were placed in a glass lined stainless steel autoclave and heated to 140°C for 8 hours. After cooling, the mixture was poured on ice and the crystalline product was filtered. The wet filter cake was dissolved in methylene chloride (100ml), dried over anyhydrous $MgSO_4$, and concentrated to dryness. The material was crystallized from hot ethanol to yield 52.8g mp (102°-106°C) (85%).

The procedure was repeated substituting phenylphosphonic dichloride (b.p.258) (3 equiv.) for phosphorous oxychloride and heating the resultant mixture at 150°C - 170°C for 4 hours. A yield of 80% - 85% of 3-cyano-2,6-dichloro-4-methylpyridine.

3. PREPARATION OF 2,6-DICHLORO-4-METHYL-3-PYRIDINECARBOXAMIDE

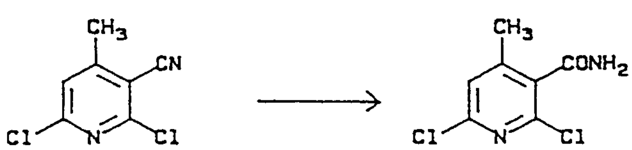

3-Cyano-2,6-dichloro-4-methylpyridine (80g, 0.42 mole) was dissolved in concentrated sulfuric acid (440ml). The mixture was heated at 150°C - 170°C with stirring for 30 minutes, cooled and poured on ice. The precipitate was filtered and washed with water. The wet filter cake was dissolved in methylene chloride, washed, neutralized with $Na_2Co_3$, dried over $Na_2SO_4$ (anhydrous), and concentrated until a white precipitate separated. Petroleum ether was added, the crystals were filtered and dried at room temperature overnight to give 83g (94%) of 2,6-dichloro-4-methyl-3-pyridinecarboxamide.

EXAMPLE I

PREPARATION OF 11-CYCLOPROPYL-5,11-DIHYDRO-4-METHYL-6H-DIPYRIDO[3,2-b:2',3'-e][1,4]-DIAZEPIN-6-ONE

A) PREPARATION OF 2,6-DICHLORO-4-METHYL-3-AMINOPYRIDINE

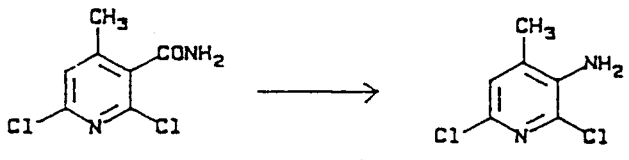

A solution of 89.2g (2.23 mole) of sodium hydroxide in 850ml of water was stirred and cooled to 0 ° C in an ice/salt bath. 34.4 g (.668 mole) of bromine was added dropwise, maintaining the temperature at 0 ° C. 120g (.585 mole) of 2,6-dichloro-4-methyl-3-pyridinecarboxamide was then added at once keeping the temperature at 0 ° C-5 ° C. The solution was slowly, over one-half hour, brought to room temperature and then heated at 70 ° C-75 ° C for one hour. The resulting suspension was cooled to room temperature, diluted further with 11 of water, and stirred overnight. The solid was filtered, back-washed with 11 of water, followed by 300 ml of petroleum ether and dried at 60 ° C to give 89.4g (86.3%) (mp: 80 ° C-83 ° C) of tan crystalline 2,6-dichloro-4-methyl-3-aminopyridine.

B) PREPARATION OF 2,6-DICHLORO-3-(2'-CHLORO-3'-NICOTINYL) CARBOXAMIDO-4-METHYLPYRIDINE

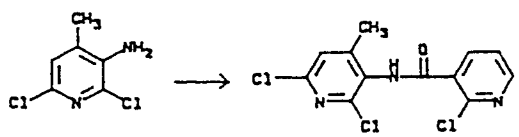

To a solution of 90.0 g (.508 mole) of 2,5-dichloro-4-methyl-3-aminopyridine in 225ml of cyclohexane, 220ml of pyridine, and 50ml of 1,4-dioxane was added in portions over 15 minutes a solution of 100.7g (.571 mole) of 2-chloronicotinic acid chloride in 200 ml of 1,4-dioxane. At the end of the addition, the reaction temperature reached 50 ° C. The mixture was stirred at room temperature for 2 days. The wet filter cake was heated at mild reflux overnight in 11 of water containing 25ml of 1N NaOH solution. (The crude product contained 8%-10% of the di-adduct, which is readily hydrolyzed in dilute sodium hydroxide solution to the required product.) The product was collected by filtration and was washed with cold water. The pure product was dried at 60 ° C under vacuum to yield 60g (37.3%) (mp:168 ° C-171 ° C) of 2,6-dichloro-3-(2'-chloro-3'-nicotinyl) carboxamido-4-methylpyridine.

2-chloronicotinic acid chloride was prepared by refluxing 90g (.571 mole) of 2-chloronicotinic acid in 315ml of thionylchloride for 2 hours. The excess thionylchloride was distilled off. The residue was diluted with 200ml of 1,4-dioxane and used as is.

C. PREPARATION OF 2,6-DICHLORO-3-(2'-CYCLOPROPYLAMINO-3'-NICOTINYL)CARBOXAMIDO-4-METHYLPYRIDINE

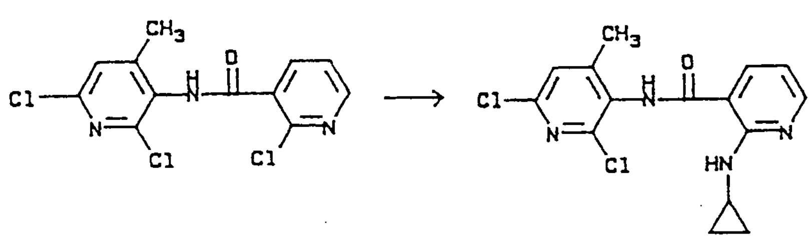

A mixture of 90g (.284 mole) of 2,6-dichloro-3-(2'-cyclopropylamino-3'-nicotinyl) carboxamide-4-methylpyridine and 79ml (1.14 mole, 4 eq.) of cyclopropylamine in 900ml of xylene was heated in a 21 stainless steel autoclave for 2 days at 90 °C-110 °C. After cooling, the xylene was concentrated to a paste and 11 of water was added. The resulting solid was filtered, back-washed with 11 of water, and 11 of petroleum ether. The product was dried at 80 °C to give 80.5g (84.1%) (mp: 163 °C-166 °C) of 2,6-dichloro-3-(2'-cyclopropylamino-3'-nicotinyl)carboxamide-4-methylpyridine.

D. PREPARATION OF 11-CYCLOPROPYL-5,11-DIHYDRO-4-METHYL-6H-DIPYRIDO[3,2-b:2',3'-e][1,4]-DIAZEPIN-6-ONE

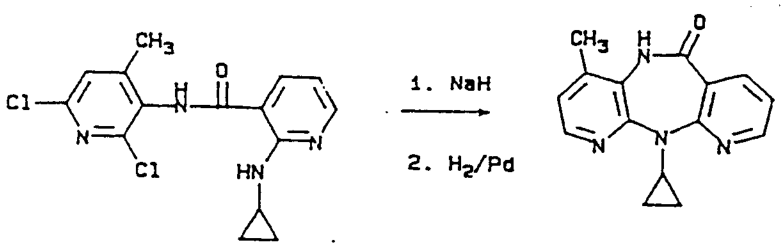


To a solution of 33g of 60% sodium hydride on mineral oil dispersion (.825 mole of sodium hydride) in 100ml of 2-methoxyethyl ether, heated to 120 °C under nitrogen, was added dropwise over .5 hour 80g (.237 mole) of 2,6-dichloro-3-(2'-cyclopropylamino-3'-nicotinyl)carboxamide-4-methylpyridine in 400 ml of 2-methoxyethyl ether. The reaction mixture was stirred at 130 °C-135 °C for 1 hour longer, cooled to room temperature, and quenched by the careful addition of ethanol. The solution was then hydrogenated as is at 50 PSI with 8g of 10% palladium on carbon catalyst for 2 days. The catalyst was filtered and the ethanol was removed under reduced pressure. The methoxyethyl ether solution was poured into 31 of water and brought to pH7 with glacial acetic acid. The crystalline product was stirred overnight, collected and washed with pentane. The filter cake was dissolved in 500ml of hot pyridine and slow addition of 31 of water gave crystalline product. After aging overnight, the product was collected and suspended in 500 ml of isopropanol, heated to reflux, cooled and filtered. Final recrystallization from pyridiine and water gave, after drying at 100 °C, 45.8g (72.5%) (mp:253 °C-254 °C) of 11-cyclopropyl-5,11-dihydro-4-methyl-6H-dipyrido-[3,2-b:2',3'-e][1,4)diazepin-6-one.

Example 2

11-Propyl-5,11-dihydro-6H-dipyrido[3,2-b:2'3'-e][1,4]diazepin-6-one

Prepared from 2,6-dichloro-3-pyridinecarboxamide, 2-chloro nicotinic acid and propylamine analogously to Example 1.

mp 184-186 °C

Example 3

11-Phenyl-5,11-dihydro-6H-dipyrido[3,2-b:2'3'-e][1,4]diazepin-6-one

Prepared from 2,6-dichloro-3-pyridine carboxamide, 2-chloro nicotinic acid and phenylamine analogously to Example 1.

mp 220-222 °C

Example 4

11-Ethyl-5,11-dihydro-4-Methyl-dipyrido[3,2-b:2'3'-e][1,4]diazepin-6-one

Prepared from 2,6-dichloro-4-methyl-3-pyridine carboxamide, 2-chloro nicotinic acid and ethylamine analogously to Example 1.

mp 212-214 °C

Example 5

11-Ethyl-5,11-dihydro-9-methyl-dipyrido[3,2-b:2'3'-e][1,4]diazepin-6-one

Prepared from 2,6-dichloro-3-pyridine carboxamide, 2-chloro-6-methyl nicotinic acid and ethylamine analogously to Example 1.

mp 244-247 °C

Example 6

11-Ethyl-3-methyl-5,11-dihydro-dipyrido[3,2-b:2'3'-e][1,4]diazepin-6-one

Prepared from 2,6-dichloro-5-methyl-3-pyridine carboxamide, 2-chloro nicotinic acid and ethylamine analogously to Example 1.

95% inhibition at 10µg/ml in the Reverse Transcripion Assay

Example 7

11-Acetyl-5,11-dihydro-dipyrido[3,2-b:2'3'-e][1,4]diazepin-6-one

Prepared from 2,6-dichloro-3-pyridine carboxamide, 2-chloro-nicotinic acid and methylcarboxamide analogously to Example 1.

>215 °C (dec.)

Example 8

11-Ethyl-5,11-dihydro-3,4-dimethyl-6H-dipyrido[3,2-b:2'3'-e] [1,4]diazepin-6-one

Prepared from 2,6-dichloro-4,5-dimethyl-3-pyridine carboxamide, 2-chloro-nicotinic acid and ethylamine analogously to Example 1.

mp 265-266 °C

Example 9

11-Ethyl-5,11-dihydro-3-chloro-6H-diypridо[3,2-b:2'3'-e][1,4] diazepin-6-one

2,5,6-trichloro-3-pyridine carboxamide, 2-chloro nicotinic acid and ethylamine analogously to Example 1.

mp 217-218 °C

Example 10

11-Ethyl-5,11-dihydro-7-methyl-6H-dipyrido[3,2-b:2'3'-e][1,4] diazepin-6-one

Prepared from 2,6-dichloro-3-pyridine carboxamide, 2-chloro-4-methyl-nicotinic acid and ethylamine analogously to Example 1.

mp 193-194 °C

Example 11

11-Ethyl-5,11-dihydro-8,9-dimethyl-6H-dipyrido[3,2-b:2'3'-e] [1,4]diazepin-6-one

Prepared from 2,6-dichloro-3-pyridine carboxamide, 2-chloro-5,6-dimethyl-nicotinic acid and ethylamine analogously to Example 1.

mp 204-206 °C

Example 12

11-Ethyl-5,11-dihydro-8-methyl-6H-dipyrido[3,2-b:2'3'-e] [1,4]diazepin-6-one

Prepared from 2,6-dichloro-3-pyridine carboxamide, 2-chloro-5-methyl-nicotinic acid and ethylamine analogously to Example 1.
mp 182-183 ° C

Example 13

11-Ethyl-5,11-dihydro-4-ethyl-6H-dipyrido[3,2-b:2'3'-e] [1,4]diazepin-6-one

Prepared from 2,6-dichloro-4-ethyl-3-pyridine carboxamide, 2-chloro nicotinic acid and ethylamine analogously to Example 1.
mp 218-219 ° C

Example 14

11-Ethyl-5,11-dihydro-8-bromo-6H-dipyrido[3,2-b:2'3'-e][1,4] diazepin-6-one

Prepared from 2,6-dichloro-3-pyridine carboxamide, 2-chloro-5-bromo-nicotinic acid and ethylamine analogously to Example 1.
mp 233.5-235.5 ° C

Example 15

11-Isopropyl-5,11-dihydro-4-methyl-6H-dipyrido[3,2-b:2'3'-e] [1,4]diazepin-6-one

Prepared from 2,6-dichloro-4-methyl-3-pyridine carboxamide, 2-chloro nicotinic acid and isopropylamine analogously to Example 1.
mp 188-189 ° C

Example 16

11-Cyclobutyl-5,11-dihydro-4-methyl-6H-dipyrido[3,2-b:2'3'-e][ 1,4]diazepin-6-one

Prepared from 2,6-dichloro-4-methyl-3-pyridine carboxamide, 2-chloro nicotinic acid and cyclobutylamine analogously to Example 1.
mp 214-215 ° C

Example 17

11-Cyclopropyl-5,11-dihydro-4-ethyl-6H-dipyrido[3,2-b:2'3'-e] [1,4]diazepin-6-one

Prepared from 2,6-dichloro-4-ethyl-3-pyridine carboxamide, 2-chloro nicotinic acid and cyclopropylamine analogously to Example 1.
mp 228-230 ° C

Example 18

11-Ethyl-5,11-dihydro-6H-dipyrido[3,2-b:2'3'-e][1,4]diazepin-6-one

Prepared from 2,6-dichloro-3-pyridine carboxamide, 2-chloro nicotinic acid and ethylamine analogously to Example 1.
mp 211-212 ° C

Example 19

11-Isopropyl-5,11-dihydro-6H-dipyrido[3,2-b:2'3'-e][1,4]diazepin-6-one

Prepared from 2,6-dichloro-3-pyridine carboxamide, 2-chloro nicotinic acid and isopropylamine analogously to Example 1.
mp 204-206 ° C

Example 20

11-Cyclopropyl-5,11-dihydro-6H-dipyrido[3,2-b:2'3'-e][1,4]diazepin-6-one

Prepared from 2,6-dichloro-3-pyridine carboxaminde, 2-chloro nicotinic acid and cyclopropylamine analogously to Example 1.
mp 240-250 ° C

Example 21

11((R)-2-Butyl)-5,11-dihydro-6H-dipyrido[3,2-b:2'3'-e][1,4] diazepin-6-one

Prepared from 2,6-dichloro-3-pyridine carboxamide, 2-chloro nicotinic acid and sec(R)butylamine analogously to Example 1.
mp 172-174 ° C

Example 22

11-((S)-2-Butyl)-5,11-dihydro-6H-dipyrido[3,2-b:2'3'-e][1,4] diazepin-6-one

Prepared from 2,6-dichloro-3-pyridine carboxamide, 2-chloro nicotinic acid and sec(S)butylamine analogously to Example 1.
mp 173-175 ° C

Example 23

11-Cyclopentyl-5,11-dihydro-6H-dipyrido[3,2-b:2'3'-e][1,4] diazepin-6-one

Prepared from 2,6-dichloro-3-pyridine carboxamide, 2-chloro nicotinic acid and cyclopentylamine analogously to Example 1.
mp 225-228 ° C

Example 24

11-Cyclopropyl-5,11-dihydro-4-methyl-7-hydroxy-6H-dipyrido[3, -b:2'3'-e][1,4] diazepin-6-one

Prepared from 2,6-dichloro-4-methyl-3-pyridine carboxamide, 2-chloro-4-hydroxy-nicotinic acid and cyclopropylamine analogously to Example 1.
mp 225-227 ° C

Example 25

11-Cyclohexyl-5,11-dihydro-6H-dipyrido[3,2-b:2'3'-e][1,4] diazepin-6-one

Prepared from 2,6-dichloro-2-pyridine carboxamide, 2-chloro nicotinic acid and cyclohexylamine analogously to Example 1.
mp 199-201 ° C

Example 26

11-Ethyl-5,11-dihydro-7,9-dimethyl-8-chloro-6H-dipyrido[3,2-b:2'3'-e][1,4] diazepin-6-one

Prepared from 2,6-dichloro-3-pyridine carboxamide, 2,5-dichloro-4,6-dimethyl-nicotinic acid and ethylamine analogously to Example 1.
mp 160-162 ° C

Example 27

11-Ethyl-5,11-dihydro-7,9-dimethyl-6H-dipyrido[3,2-b:2'3'-e] [1,4] diazepin-6-one

Prepared from 2,6-dichloro-3-pyridine carboxamide, 2-chloro-4,6-dimethyl-nicotinic acid and ethylamine analogously to Example 1.
mp 245-247 ° C

Example 28

11-Cyclobutyl-5,11-dihydro-6H-diyprido[3,2-b:2'3'-e][1,4] diazepin-6-one

Prepared from 2,6-dichloro-3-pyridine carboxamide, 2-chloronicotinic acid and cyclobutylamine analogously to Example 1.
mp 241-243 ° C

## Claims

**1.** A method for the preparation of a compound of the formula:

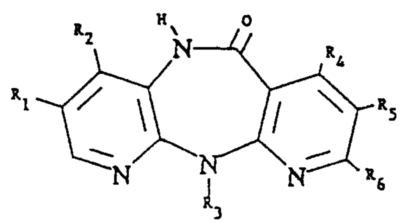

(I)

wherein $R_1$ is hydrogen, alkyl of 1 to 4 carbon atoms, alkoxy or alkylthio of 1 to 4 carbon atoms, alkoxycarbonyl of 2 to 4 carbon atoms, trihalomethyl, hydroxyalkyl of 1 to 4 carbon atoms, alkanoyl of 2 to 4 carbon atoms, alkanoyloxy of 2 to 4 carbon atoms, alkanoylamino of 1 to 4 carbon atoms, aminoalkyl of 1 to 4 carbon atoms, alkoxycarbonylalkyl wherein the alkoxy and alkyl moieties each contain 1 to 2 carbon atoms, carboxyalkyl of 2 to 4 carbon atoms, mono- or di-alkylamino wherein each alkyl moiety contains 1 to 2 carbon atoms, cyano, nitro, hydroxyl, carboxy, amino, mono- or di-alkylaminoalkyl wherein each alkyl moiety contains 1 to 2 carbon atoms, azido or halogen;

$R_2$ is hydrogen or alkyl of 1 to 3 carbon atoms, with the proviso that if $R_1$ is other than hydrogen or alkyl of 1 to 3 carbon atoms, then $R_2$ is hydrogen;

$R_3$ is alkyl or fluoroalkyl of 1 to 5 carbon atoms, cycloalkyl of 3 to 6 carbon atoms, alkenyl or alkynyl of 2 to 5 carbon atoms, alkoxyalkyl or alkylthioalkyl of 2 to 4 carbon atoms, alkanoyl of 2 to 4 carbon atoms, hydroxylakyl of 2 to 4 carbon atoms, arylmethyl (wherein the aryl moiety is phenyl, thienyl or furanyl, which is either unsubstituted or substituted by alkyl or alkoxy of 1 to 3 carbon atoms, hydroxyl or halogen), phenyl (which is either unsubstituted or substituted by alkyl or alkoxy of 1 to 3 carbon atoms, hydroxy or halogen), or alkoxycarbonylmethyl wherein the alkoxy moiety contains 1 to 5 carbon atoms;

one of $R_4$, $R_5$ and $R_6$ is alkyl of 1 to 4 carbon atoms, alkenyl or alkynyl of 2 to 4 carbon atoms, trihalomethyl, hydroxyalkyl of 1 to 4 carbon atoms, alkloxyalkyl or alkylthioalkyl of 2 to 4 carbon atoms,

alkyloxycarbonylalkyl wherein the alkyl moieties each contain 1 to 2 carbon atoms, hydroxyl, alkyloxy or alkylthio of 1 to 4 carbon atoms, hydroxyalkyloxy of 2 to 4 carbon atoms, alkanoyloxy of 2 to 4 carbon atoms, alkylsulfinyl or alkylsulfonyl of 1 to 4 carbon atoms, alkanyol of 2 to 6 carbon atoms, alkoxycarbonyl wherein the alkyl moiety contains 1 to 3 carbon atoms, aminoalkyl of 1 to 4 carbon atoms, mono- or di-alkylaminoalkyl wherein each alkyl moiety contains 1 to 2 carbon atoms, halogen, cyano, nitro, azido or carboxyl, with the other two substituents being hydrogen; or,

two of $R_4$, $R_5$ and $R_6$ are independently alkyl of 1 to 2 carbon atoms, trihalomethyl, alkyloxy or alkylthio of 1 to 2 carbon atoms, or halogen, with the remaining substituent being hydrogen; or

$R_4$, $R_5$ and $R_6$ are each hydrogen,

which method comprises the following steps:

a) reacting a compound having the formula

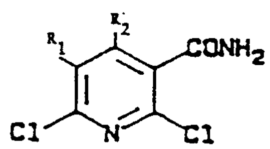

(II)

in an aqueous solution, with a base and a halide, at about 0 ° C to about 100 ° C, to produce a compound having the formula

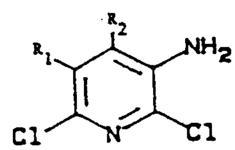

(III)

b) reacting the compound of formula III prepared in a), with a compound of the formula

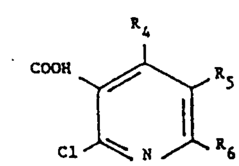

wherein $R_4$, $R_5$, and $R_6$ are as defined above, in an anhydrous organic solvent, between about 20 ° C to about 80 ° C, to produce a compound having the formula

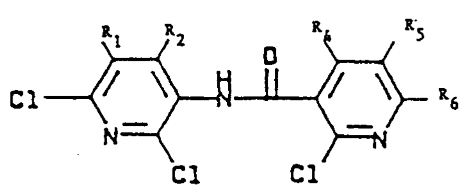

(IV)

c) reacting the compound of formula IV prepared in b), with a compound of the formula $R_3NH_2$, in an inert organic solvent at between about 20 ° C to about 150 ° C, to produce a compound of the formula

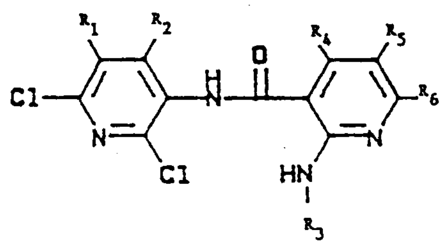

(V)

d) heating the compound of formula V prepared in c), in an inert organic solvent comprising sodium hydride at between about 60 ° C to about 150 ° C; then hydrogenating the resultant solution in the presence of a metal catalyst, between about 20 ° C to about 100 ° C, to produce a mixture comprising the compound of formula I; and then isolating the compound of formula I from the mixture as such or as a salt thereof.

**2.** A method as recited in claim 1 wherein $R_1$ and $R_2$ are each hydrogen or alkyl of 1 to 3 carbon atoms.

**3.** A method as recited in claim 2 wherein $R_3$ is alkyl of 1 to 5 carbon atoms or cycloalkyl of 3 to 6 carbon atoms.

**4.** A method as recited in claim 3 wherein $R_4$ , $R_5$ $R_6$ are each hydrogen.

**5.** A method as recited in claim 4 wherein $R_3$ is cycloalkyl of 3 to 6 carbon atoms.

**6.** A method as recited in claim 5 wherein $R_1$ is hydrogen.

**7.** A method according to claim 6, wherein the compound 11-cyclopropyl-5,11-dihydro-4-methyl-6H-dihydro[3,2-b:2'3'-e][1,4]diazepin-6-one or a salt thereof is produced.

European Patent Office

# EUROPEAN SEARCH REPORT

Application Number

**EP 91 11 7599**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,P | EP-A-0 410 148 (BOEHRINGER INGELHEIM PHARMACEUTICALS INC. / DR. K. THOMAE GMBH)<br>* page 3, line 34 - page 4, line 15 *<br>* page 6, line 46 - page 7, line 32 * | 1 | C 07 D 471/14 //<br>(C 07 D 471/14<br>C 07 D<br>C 07 D 243:00<br>C 07 D 221:00<br>C 07 D<br>C 07 D 221:00 ) |
| A,P | EP-A-0 393 529 (BOEHRINGER INGELHEIM PHARMACEUTICALS INC. / DR. K. THOMAE GMBH)<br>* page 3, lines 2 - 29 *<br>* page 6, lines 19 - 58 * | 1 | |
| A | EP-A-0 066 774 (DR. K. THOMAE GMBH)<br>* pages 1, 2; page 4 to page 5, line 11 * | 1 | |
| A | DE-A-3 222 809 (DR. K. THOMAE GMBH)<br>* claim 1 * | 1 | |
| A | DE-A-2 424 811 (DR. K. THOMAE GMBH)<br>* page 2, compound II *<br>* page 4, last paragraph to page 6, line 2 * | 1 | |
| A | DE-A-3 208 656 (CRC S.P.A.)<br>* page 5, scheme 1 * | 1 | **TECHNICAL FIELDS SEARCHED (Int. Cl.5)**<br>C 07 D 471/00 |

The present search report has been drawn up for all claims

| Place of search | Date of completion of search | Examiner |
|---|---|---|
| Berlin | 13 January 92 | HASS C V F |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document